(19) Europäisches Patentamt
European Patent Office
Office européen des brevets

(11) Veröffentlichungsnummer : **0 360 784 B1**

# (12) EUROPÄISCHE PATENTSCHRIFT

(45) Veröffentlichungstag der Patentschrift : **02.12.92 Patentblatt 92/49**

(51) Int. Cl.$^5$ : **C07D 409/06,** A61K 31/38

(21) Anmeldenummer : **89890235.8**

(22) Anmeldetag : **12.09.89**

## (54) Thienyloxy-alkylamin-Derivate, Verfahren zu ihrer Herstellung und diese Verbindungen enthaltende Arzneimittel.

(30) Priorität : **15.09.88 AT 2266/88**

(43) Veröffentlichungstag der Anmeldung : **28.03.90 Patentblatt 90/13**

(45) Bekanntmachung des Hinweises auf die Patenterteilung : **02.12.92 Patentblatt 92/49**

(84) Benannte Vertragsstaaten : **AT BE CH DE ES FR GB GR IT LI LU NL SE**

(56) Entgegenhaltungen :
**EP-A- 0 266 336**
**FR-A- 2 600 065**

(73) Patentinhaber : **EBEWE Arzneimittel Gesellschaft mbH**
**A-4866 Unterach (AT)**

(72) Erfinder : **Binder, Dieter, Dr.**
**Sieveringerstrasse 207**
**A-1190 Wien (AT)**
Erfinder : **Greier, Gerhard, Dr. Dipl.-Ing.**
**Huemerstrasse 21/8**
**A-4020 Linz (AT)**
Erfinder : **Rovenszky, Franz, Dr. Dipl.-Ing.**
**Lagerhausstrasse 5/8**
**A-2460 Bruck an der Leitha (AT)**
Erfinder : **Hillebrand, Friedrich, Dr. Dipl.-Ing.**
**Hugo-Wolf-Weg 355**
**A-4866 Unterach am Attersee (AT)**

(74) Vertreter : **Pfeifer, Otto, Dipl.-Ing. et al**
**Fleischmanngasse 9**
**A-1040 Wien (AT)**

**EP 0 360 784 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

In der europäischen Patentveröffentlichung EP-A1-O 266 336 werden 1-[3-(2-Di-alkylaminoethoxy)-2-thienyl]-3-phenyl-1-propanone beschrieben, die zur Behandlung von Herzrhythmusstörungen verwendbar sind.

Die Verbindungen der vorliegenden Erfindung unterscheiden sich von diesen bekannten Verbindungen im wesentlichen durch das Vorhandensein einer Benzofurangruppe an Stelle eines Phenethylsubstituenten.

Die Erfindung betrifft neue therapeutisch wertvolle Thienyloxyalkylamin-Derivate der allgemeinen Formel

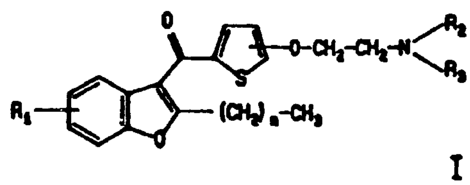

I ,

wobei $-O-CH_2-CH_2-N(R_2,R_3)$ in Position 4 oder 5 des Thiophenrings stehen kann und worin

$R_1$ Wasserstoff, Halogen, $CF_3$, Alkyl oder Alkoxy darstellt,

$R_2$ und $R_3$ gleich oder verschieden sind und jeweils Alkyl, Cycloalkyl, Alkenyl oder Alkinyl mit jeweils bis zu 8 C-Atomen bedeuten oder $R_2$ und $R_3$ zusammen mit dem sie verbindenden Stickstoffatom einen 5 bis 7-gliedrigen gesättigten Ring oder einen gesättigten heterocyclischen Ring bilden, der gegebenenfalls ein Sauerstoff- oder Stickstoffatom als weiteres Heteroatom im Ring enthalten kann, wobei ein zusätzliches Stickstoffatom durch einen Alkylrest mit 1 bis 3 C-Atomen substituiert sein kann, und

n eine ganze Zahl 1 bis 5 bedeutet,

und ihre pharmazeutisch verträglichen Säureadditionssalze.

Die neuen Thienyloxy-alkylamin-Derivate weisen eine vorteilhafte antiarrhythmische Wirkung auf und finden insbesondere Einsatz bei Erkrankungen des Herz-Kreislaufsystems.

Eine bevorzugte Klasse von Verbindungen der allgemeinen Formel I ist jene, worin $R_1$ = H und n = 3 bedeuten.

Die Erfindung bezieht sich weiterhin auf ein Verfahren zur Herstellung der neuen Verbindungen der allgemeinen Formel I, welches dadurch gekennzeichnet ist, daß man eine Verbindung der allgemeinen Formel

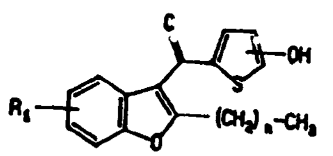

II,

wobei OH in Position 4 oder 5 des Thiophenringes stehen kann und in der $R_1$ und n die obige Bedeutung haben, mit einer Verbindung der allgemeinen Formel

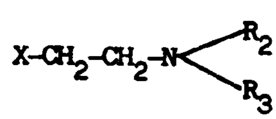

III,

in der $R_2$ und $R_3$ obige Bedeutung haben und X Chlor, Brom oder Jod bedeutet, in Gegenwart von mindestens einem Äquivalent einer starken Base in einem inerten organischen Lösungsmittel bei einer Temperatur von 60 bis 120°C umsetzt und die erhaltenen Basen der Formel I gegebenenfalls in die Säureadditionssalze überführt.

Die erfindungsgemäße Umsetzung wird am besten so durchgeführt, daß man eine Verbindung der Formel II in einem inerten organischen Lösungsmittel, wie z.B. Dimethylformamid (DMF), Dimethylsulfoxid (DMSO), oder Diethylcarbonat, löst und mit mindestens einem Äquivalent einer starken Base, vorzugsweise einem Alkalialkoholat oder -hydrid, versetzt und im Falle eines Alkalialkoholats den entstehenden oder als Lösungsmittel des Alkoholats dienenden Alkohol abdestilliert. Die Umsetzungstemperatur liegt dabei zwischen 60 und 120°C. Anschließend erfolgt die Umsetzung mit einer Verbindung der allgemeinen Formel III bei einer Temperatur zwischen 50 und 100°C. Die Reaktionszeit beträgt dann zwischen 30 Minuten und 2 Stunden.

Da die freien Basen der allgemeinen Formel I meist nur schwer kristallisierende und meist nicht unzersetzt destillierbare Öle sind, empfiehlt es sich, die Reinigung über gut kristallisierende Säureadditionsverbindungen, wie z.B. die Hydrochloride, vorzunehmen, die man gut umkristallisieren kann.

Dazu löst man die rohe Base in einem geeigneten Lösungsmittel, z.B. in einem niederen Alkohol oder Ether, gibt eine mindestens äquivalente Menge Protonensäure zu, dampft das Lösungsmittel im Vakuum ab und kristallisiert den Rückstand aus Methanol, Ethanol oder vorzugsweise Aceton, gegebenenfalls unter Zusatz von Ether, um.

Diese Säureadditionssalze können dann in an sich bekannter Weise, z.B. mit Alkalien oder Ionenaustauschern, in die freien Verbindungen übergeführt werden, von denen sich durch Umsetzung mit anorganischen oder organischen Säuren, insbesondere solchen, die zur Bildung von therapeutisch verwendbaren Salzen geeignet sind, weitere Salze gewinnen lassen.

Infolge der engen Beziehungen zwischen den neuen Verbindungen und deren Salzen sind im vorausgegangenen und nachfolgenden sinn- und zweckmäßig gegebenenfalls auch unter den freien Basen die entsprechenden Salze zu verstehen.

Die Verbindungen der allgemeinen Formel II können, ausgehend von den literaturbekannten Substanzen der Formel IV, in der $-OCH_3$ in Position 4 (S. Gronowitz, Ark. Kemi, 12, 239 (1958) oder 5 (J. Sice, J.Am. Chem.Soc., 75, 3697 (1953) des Thiophenringes stehen kann, und den literaturbekannten Verbindungen der Formel VI (z.B. N.P. Buu-Hoi, N.D. Xuong u. N.V. Bac, J.Chem.Soc.1964,173), in der $R_1$ und n die obige Bedeutung haben, gemäß dem folgenden Reaktionsschema nach üblichen und dem Fachmann geläufigen chemischen Arbeitsmethoden hergestellt werden:

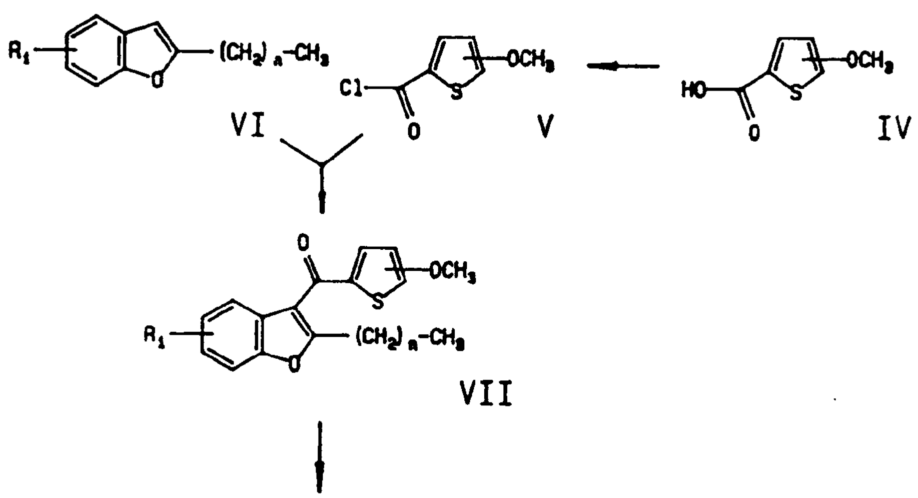

VI V IV

VII

II

Die Verbindungen der Formel III sind, soweit sie nicht literaturbekannt oder käuflich sind, auf dem Fachmann bekannter Weise, ausgehend von den literaturbekannten Verbindungen VIII, herstellbar, z.B.

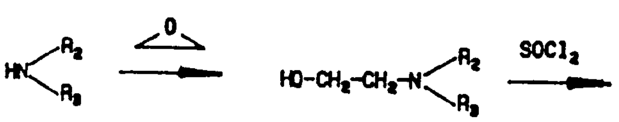


III.HCl

VIII IX

Die Säureadditionssalze der Endverbindungen können in an sich bekannter Weise, beispielsweise durch Zusetzen eines Alkalis oder durch Ionenaustauscher, in die freien Basen übergeführt werden. Andere Salze können daraus durch Umsetzen mit anorganischen oder organischen Säuren, insbesondere solchen, die zur Bildung von therapeutisch verwendbaren und pharmazeutisch annehmbaren Salzen geeignet sind, gebildet werden.

Geeignete Beispiele für derartige pharmazeutisch verträgliche Salze sind neben dem Salz der Salzsäure das der Bromwasserstoffsäure, der Schwefelsäure, der Salpetersäure, der Phosphorsäure, von Sulfonsäuren,

der Essigsäure, der Benzoesäure, der Maleinsäure, der Weinsäure, der Zitronensäure und dgl. Es können jedoch auch andere Säuren verwendet werden.

Die neuen Verbindungen der Formel I und ihre pharmazeutisch verwendbaren Salze zeigen hervorragende antiarrhytmische Eigenschaften.

Auf Grund dieser pharmakologischen Eigenschaften können die neuen Verbindungen allein oder in Mischung mit anderen Wirksubstanzen in Form üblicher galenischer Zubereitungen bei Erkrankungen, die durch Herzrhythmusstörungen,wie z.B. Tachykardie, verursacht werden, als Medikament verwendet werden.

Unter den Arten von Tachykardien, die mit den erfindungsgemäßen Verbindungen behandelt werden können, seien supraventrikuläre Tachykardie, ventrikuläre Tachykardie, ventrikuläre Ektopie und "reentry" Tachykardie genannt.

Die Erfindung bezieht sich weiterhin auf Heilmittel, die z.B. in Form pharmazeutischer Präparate Verwendung finden, welche die erfindungsgemäßen Verbindungen der allgemeinen Formel I in Mischung mit einem für die enterale oder parenterale Applikation geeigneten, pharmazeutischen, organischen oder anorganischen Trägermaterial,beispielsweise Wasser, Gelatine, Gummi arabicum, Milchzucker, Stärke, Magnesiumstearat, Talk, pflanzliche Öle, Polyalkylenglykole, Vaseline oder dergleichen enthalten.

Die pharmazeutischen Präparate können in fester Form z.B. als Tabletten, Filmtabletten,Dragees, Suppositorien, Kapseln, Mikrokapseln oder in flüssiger Form z.B. als Lösungen, Injektionslösungen, Suspensionen oder Emulsionen oder in Zusammensetzungen mit verzögerter Freigabe des Wirkstoffes vorliegen.

Gegebenenfalls sind sie sterilisiert und/oder enthalten Hilfsstoffe, wie Konservierungs-, Stabilisierungs- oder Emulgiermittel, Salze zur Veränderung des osmotischen Druckes oder Puffer.

Insbesondere können pharmazeutische Präparate die erfindungsgemäßen Verbindungen in Kombination mit anderen therapeutisch wertvollen Stoffen enthalten. Mit diesen können die erfindungsgemäßen Verbindungen zusammen mit den oben angegebenen Hilfs- und/oder Trägerstoffen zu Kombinationspräpraraten formuliert werden.

Die neuen Verbindungen können in den erfindungsgemäßen pharmazeutischen Zusammensetzungen in einem Anteil von etwa 20 bis 200 mg/Tablette vorhanden sein, wobei der Rest ein pharmazeutisch annehmbarer Füllstoff ist.

Eine geeignete Dosis zur Verabreichung der neuen Verbindungen beträgt etwa 2 bis 20 mg/kg pro Tag, jedoch kommen je nach dem Zustand des zu behandelnden Patienten auch andere Dosen in Frage. Die neuen Verbindungen können in mehreren Dosen und auf oralem Weg verabreicht werden.

Die neue Verbindung [3-(2.Butyl-benzo[b]furanyl)]-[5-(2-diethylaminoethoxy-2-thienyl)]-methanon, Hydrochlorid als repräsentative Verbindung für die neue Substanzklasse wurde sowohl in vivo als auch in vitro auf ihre antiarrhythmischen Eigenschaften getestet. Am isolierten perfundierten Meerschweinchenherz nach Langendorf zeigt die Substanz einen deutlich antiarrhythmischen Effekt mit einem ausgeprägten natriumantagonistischen Anteil. Die Substanz ist in deutlich niedrigerer Konzentration als das Vergleichs-Antiarrhythmikum Amiodaron wirksam.

Gegenüber den bestehenden Antiarrhythmika der Klasse 1 zeigt sich der Vorteil, daß eine gleichmäßige Verlängerung der Leitzeiten und der Refraktärzeiten des Herzens erfolgt, daher ist auch keine arrhythmogene Potenz vorhanden. Bei dem Modell der Reperfusionsarrhythmie am narkotisierten Hund ist die Verbindung in einer Dosierung von 2 mg pro kg Körpergewicht bei ventrikulären Arrhythmien wirksam und führt diese in einen Sinusrhythmus über. Gegenüber herkömmlichen Antiarrhythmika der Klasse 1C erfolgt keine Beeinflussung der Hämodynamik.

Die folgenden Beispiele erläutern die Erfindung näher, ohne daß diese hierauf beschränkt sein soll.

## BEISPIEL 1:

### [3-(2-Butyl-benzo[b]furanyl)]-[5-(2-diethylaminoethoxy)-2-thienyl]-methanon, Hydrochlorid

Zu einer Lösung von 7,.1 g (23,7 mmol) (2-Butyl-3-benzo|b|furanyl)-(5-hydroxy-2-thienyl)-methanon in 140 ml Diethylcarbonat werden 29,5 ml einer 1 molaren methanolischen Natriummethylatlösung zugegeben und anschließend in ein 132°C heißes Ölbad getaucht. Es wird solange MeOh abdestilliert, bis das Reaktionsgemisch eine Temperatur von 110 bis 112°C erreicht. Danach wird abgekühlt und bei einer Temperatur von 35 bis 40°C eine Lösung von 4,1 g (30,2 mmol) 2-Diethylaminoethylchlorid (hergestellt durch Verteilen von 5,8 g 2-Diethylaminoethylchlorid, Hydrochlorid (FLUKA Art. Nr. 31810) zwischen 100 ml ges.Natriumhydrogencarbonatlösung und 40 ml Ether, dreimalige Extraktion der wäßrigen Phase mit je 30 ml Ether, Trocknen und Eindampfen der vereinigten organischen Phasen) in 57 ml Diethylcarbonat zugegeben. Nach beendeter Zugabe wird auf 90 bis 93°C erwärmt. Aus der Suspension fällt ein feiner gelber Niederschlag aus. Nach 50 Minuten wird eingedampft, der schmierig kristalline Rückstand zwischen 350 ml einer ges. Natriumhydrogencarbonatlösung und 200 ml Ether verteilt, gerührt und die Phasen getrennt. Die $H_2O$-Phase wird noch dreimal mit Ether ausgeschüttelt und die vereinigten organischen Phasen über Natriumsulfat/Aktivkohle getrocknet und eingedampft. Es werden 9,0 g eines orangen Öles (95 % d.Th.) erhalten.

Das Rohprodukt wird in 120 ml abs. Ether gelöst und unter Kühlung trockenes HCl-Gas eingeleitet. Das zunächst leicht schmierig anfallende Hydrochlorid wird kristallisiert und die hellgelben Kristalle abgesaugt. Die so erhaltenen ca. 10 g Rohprodukt werden in 180 ml abs. Aceton gelöst, filtriert und die Lösung auf 100 ml eingeengt. Es wird abgekühlt und die Kristallisation über Nacht im Tiefkühlschrank vervollständigt.

Die ausgefallenen Kristalle werden abgesaugt und mit kaltem Aceton gewaschen.

Ausbeute: 7,5 g farblose Kristalle (73 % d.Th.)
Fp.: 130 - 132°C
Mikroelementaranalyse: $C_{23}H_{29}NO_3S$ (436.02)

| | C | H | N |
|---|---|---|---|
| berechnet: | 63,36 | 6,94 | 3,21 |
| gefunden: | 63,22 | 6,93 | 3,15 |

$^1$H-NMR: ($CDCl_3$):
δ(ppm): 13-11 (sehr breit; 1H; HCl); 7,61 - 7,19 (m; 5H; 4 Bz-H u. Th-$H_3$); 6,37 (d; 1H; Th-$H_4$); 4,79 (t; 2H; -$OCH_2$); 3,52 (t; 2H; -$NCH_2$); 3,28 (q; 4H; -$N(CH_2$-$CH_3)_2$); 2,96 (t; 2H; Ar-$CH_2$); 1,90 - 1,25 (m; 4H; -$CH_2$-$CH_2$-); 1,46 (t; 6H; -$N(CH_2$-$CH_3)_2$; 0,91 (t; 3H; -$CH_3$).

Das Ausgangsprodukt kann wie folgt hergestellt werden:

5-Methoxy-2-thiophencarbonsäurechlorid

15,0 g (94,8 mmol) 5-Methoxy-2-thiophencarbonsäure werden in 100 ml Thionylchlorid und 1 ml abs.DMF 30 Minuten unter Rückfluß erhitzt. Anschließend wird das überschüssige Thionylchlorid im Vakuum abdestilliert. Das Rohprodukt (ca. 18 g braünliches Öl) wird kurzwegdestilliert.

Ausbeute: 14,6 g gelbliches Öl (87 % d.Th.)
Siedepunkt: 70 - 75°C/0,04 mbar

(2-Butyl-3-benzo[b]furanyl)-(5-methoxy-2-thienyl)-methanon

3,0 g (17,2 mmol) 2-Butyl-benzo[b]furan werden in 30 ml abs.Chloroform gelöst, die Lösung auf 0°C gekühlt und mit 3,6 g (20,4 mmol) 5-Methoxy-2-thiophencarbonsäurechlorid, gelöst in 5 ml abs. Chloroform, versetzt und anschließend bei einer Temperatur zwischen 0 und 3°C 2,8 ml $SnCl_4$ zugetropft. Es wird noch 2 Stunden bei 0°C gerührt.Das Reaktionsgemisch wird unter Eiskühlung auf 50 ml 2n HCl geleert, die Phasen getrennt und die wäßrige Phase mit Ether erschöpfend extrahiert. Die Chloroform- und die Etherphase werden mit jeweils 50 ml 2n NaOH gewaschen, vereinigt, über Natriumsulfat getrocknet und eingedampft. Das Rohprodukt wird kurzwegdestilliert.

Ausbeute: 4,4 g hellgelbes Öl (81 % d.Th.)
Siedepunkt: 145 - 148°C/0,03 mbar

(2-Butyl-3-benzo[b]furanyl)-(5-hydroxy-2-thienyl)-methanon

Eine Lösung von 10,0 g (31,8 mmol) (2-Butyl-3-benzo[b]furanyl)(5-methoxy-2-thienyl)-methanon in 100 ml abs. Chloroform wird auf 15°C gekühlt und bei einer Temperatur zwischen 15 und 20°C mit 10 ml (106 mmol) Bortribromid versetzt. Anschließend wird das Reaktionsgemisch unter Rückfluß erhitzt.

Nach 1,5 Stunden wird auf 300 ml 2n HCl geleert, 10 Minuten heftig gerührt, die Phasen getrennt und die wäßrige noch 2 mal mit Methylenchlorid extrahiert. Die vereinigten organischen Phasen werden 5 mal mit je 120 ml ges.Natriumhydrogencarbonatlösung ausgeschüttelt. Die vereinigten wäßrigen Phasen werden mit conc. HCl angesäuert und mit Methylenchlorid extrahiert. Die vereinigten organischen Phasen werden über Natriumsulfat/Aktivkohle getrocknet, filtriert und eingedampft. Das Rohprodukt wird direkt in die nächste Stufe eingesetzt.

Ausbeute: 7,6 g zähes Öl (80 % d.Th.)
Mikroelementaranalyse: $C_{17}H_{16}O_3S$ (300,21)

| | C | H |
|---|---|---|
| berechnet: | 67,98 | 5,37 |
| gefunden: | 68,04 | 5,42 |

$^1$H-NMR: (In $CDCl_3$ liegt die Verbindung in 3 tautomeren Formen a, b und c vor)

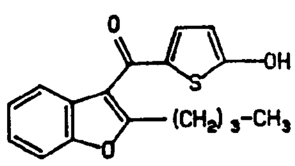
a

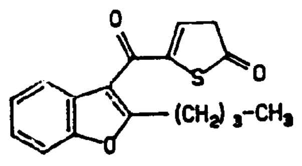
b

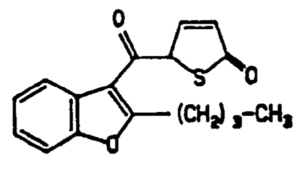
c

a: δ(ppm): 7,60 - 7,15 (m; 4H; Bz-H); 7,42 (d; 1H; Th-$H_3$); 6,18 (d; 1H; Th-$H_4$); 2,85 (t; 2H; Ar-$CH_2$); 1,98-1,10 (m; 4H; -$CH_2$-$CH_2$); 0,90 (t; 3H; -$CH_3$).
b: δ(ppm): 7,60 - 7,15 (m; 4H; Bz-H); 6,66 (dd; 1H; Th-$H_3$); 3,76 (d; 2H; Th-$H_4$); 2,85 (t; 2H; Ar-$CH_2$) 1,98-1,10 (m; 4H; -$CH_2$-$CH_2$); 0,90 (t; 3H;-CH3)
c: δ(ppm): 7,70 (dd; 1H; Th-$H_4$) 7,60 - 7,15 (m; 4H; Bz-H); 6,45 (dd; 1H; Th-$H_3$); 5,88 (dd; 1H; Th-$H_2$); 2,85 (t; 2H; Ar-$CH_2$); 1,98 - 1,10 (m; 4H; -$CH_2$-$CH_2$); 0,90 (t; 3H; -$CH_3$).

BEISPIEL 2:

[3-(2-Butyl-benzo[b]furanyl)]-[5-(2-(1-pyrrolidinyl)-ethoxy)-2-thienyl]-methanon, Hydrochlorid

6,00 g (20,0 mmol) [3-(2-Butyl-benzo[b]furanyl)]-(5-hydroxy-2-thienyl)-methanon werden in 120 ml Diethylcarbonat gelöst und mit 4,60 ml 5,6 M Natriummethylatlösung versetzt. Anschließend wird in ein 135°C heißes Ölbad eingesenkt und unter Stickstoffstrom Methanol abdestilliert, bis der Siedepunkt des Reaktionsgemisches bei 115°C liegt. Danach wird abgekühlt und bei einer Temperatur von 35 bis 40°C wird eine Lösung von 3,50 g (26,3 mmol) 1-(2-Chlorethyl)-pyrrolidin (freigesetzt aus 4,50 g Hydrochlorid, FLUKA Art. Nr. 23065) in 60 ml Diethylcarbonat zugetropft. Nach beendeter Zugabe wird auf 90 - 95°C erwärmt. Nach einer Stunde wird eingedampft, der schmierig-kristalline Rückstand zwischen 300 ml gesättigter Natriumhydrogencarbonatlösung und 200 ml Ether verteilt, verührt und die Phasen getrennt. Die Wasserphase wird noch dreimal mit insgesamt 300 ml Ether ausgeschüttelt und die vereinten organischen Phasen über Natriumsulfat getrocknet und eingedampft. Es werden 6,73 g eines braunen Öles erhalten.

Dieses Produkt wird in 50 ml 4n Salzsäure suspendiert und 10 Minuten unter Rückfluß erhitzt. Danach wird mit 4n Natronlauge neutralisiert und fünfmal mit insgesamt 500 ml Ether extrahiert. Die vereinten organischen Phasen werden über Natriumsulfat getrocknet, filtriert und eingedampft.

Das Rohprodukt wird in 100ml abs.Ether aufgenommen und unter Kühlung trockener Chlorwasserstoff eingeleitet. Das kristallin ausfallende Produkt wird abgesaugt und aus Ethylacetat umkristallisiert.
Ausbeute: 7,00 g farblose Kristalle (80,7% d.Th.)
Fp: 137 - 139°C (Ethylacetat)
Mikroelementaranalyse: $C_{23}H_{28}NC1O_3S$ (434,00)

| | C | H | N |
|---|---|---|---|
| berechnet: | 63,65 | 6,50 | 3,23 |
| gefunden: | 63,42 | 6,54 | 3,17 |

$^1$H-NMR: ($CDCl_3$)

δ(ppm). 7,56 - 7,19 (m, 5H, Bz-H und Th-$H_3$); 6,37 (d, 1H, Th-$H_4$); 4,75 (t, 2H, -O-$CH_2$-); 3,80 - 3,50 (m, 6H, -$CH_2$-N(-$CH_2$)$_2$); 2,96 (t, 2H, Ar-$CH_2$-); 2,10 (m, 4H, Pyr: -$CH_2$-$CH_2$-); 1,90 - 1,30 (m, 4H, -$CH_2$-$CH_2$-$CH_3$); 0,91 (t, 3H, -$CH_3$).

BEISPIEL 3:

[3-(2-Butyl-benzo[b]furanyl)]-[4-(2-diethylaminoethoxy)-2-thienyl]-methanon, Hydrochlorid

3,50 g (11,7 mmol) [3-(2-Butyl-benzo[b]furanyl)]-(4-hydroxy-2-thienyl)-methanon werden in 70 ml Diethylcarbonat gelöst und mit 2,70 ml einer 30%igen Natriummethylatlösung versetzt. Anschließend wird in ein 135°C heißes Ölbad eingesenkt und Methanol im Stickstoffstrom abdestilliert, bis nach 3 Stunden der Siedepunkt des Reaktionsgemisches bei 120°C liegt. Danach wird abgekühlt und bei einer Temperatur von 35 bis 40°C wird eine Lösung von 1,74 g (12,8 mmol) 2-Diethylaminoethylchlorid (freigesetzt aus 2,20 g Hydrochlorid, FLUKA Art. Nr. 31810) in 20 ml Diethylcarbonat zugetropft. Nach beendeter Zugabe wird auf 90 bis 95°C erwärmt und über Nacht gerührt. Das Lösungsmittel wird abdestilliert, der Rückstand zwischen 100 ml gesättigter Natriumhydrogencarbonatlösung und 100 ml Ether verteilt, gerührt und die Phasen getrennt. Die wäßrige Phase wird noch dreimal mit insgesamt 200 ml Ether extrahiert und die vereinten organischen Phasen über Natriumsulfat getrocknet und eingedampft.

Der Rückstand (3,20g braunes Öl) wird säulenchromatographisch gereinigt (100 g Kieselgel, Eluens: Ethylacetat). Es werden 1,95 g gelbes Öl erhalten.

Diese wird in 50 ml abs.Ether aufgenommen und Chlorwasserstoff bis zur Sättigung eingeleitet. Das zunächst schmierig anfallende Hydrochlorid wird durch Reiben zur Kristallisation gebracht und die hellgelben Kristalle abgesaugt. Das so erhaltene Rohprodukt wird aus 20 ml Ethylacetat umkristallisiert.
Ausbeute: 1,52 g hellgelbe, hygroskopische Kristalle (29,9 % d.Th.)
Fp: 70 - 71°C (Ethylacetat)
Mikroelementaranalyse: $C_{23}H_{30}NClO_3S.1,1\ H_2O$ (455,83)

| | C | H | N |
|---|---|---|---|
| berechnet: | 60,60 | 7,12 | 3,07 |
| gefunden: | 60,45 | 6,80 | 3,14 |

$^1$H-NMR: ($CDCl_3$)
δ(ppm): 7,47 - 6,92 (m, 5H, Bz-H und Th-$H_3$); 6,96 (d, 1H, Th-$H_5$); 4,95 (t, 2H, -O-$CH_2$-); 3,52 - 3,00 (m, 6H, -$CH_2$-N(-$CH_2$)$_2$); 3,00 - 2,52 (m, 5H, Ar-$CH_2$- +HCl + $H_2O$); 1.94 - 1,42 (m, 4H, -$CH_2$-$CH_2$); 1,42 (t, 6H, -N-$CH_2$-$CH_3$); 0,91 (t, 3H, -$CH_3$).

Das Ausgangsprodukt kann wie folgt hergestellt werden:

4-Methoxy-2-thiophencarbonsäurechlorid:

24,0 g (152 mmol) 4-Methoxy-2-thiophencarbonsäure werden in 250 ml Thionylchlorid suspendiert und zwei Stunden unter Rückfluß erhitzt. Anschließend wird das überschüssige Thionylchlorid im Vakuum abdestilliert. Das Rohprodukt wird kurzwegdestilliert und kristallisiert in der Vorlage aus.
Ausbeute: 20,0 g hellgelbe Kristalle (74,6 % d.Th.)
Fp: 40°C
Siedepunkt: 122 - 124°C/15 mbar

[3-(2-Butyl-benzo[b]furanyl)]-(4-methoxy-2-thienyl)-methanon

15,0 g (87,1 mmol) 2-Butyl-benzo[b]furan werden in 75 ml abs.Chloroform gelöst und bei Raumtemperatur mit 18,6 g (0,11 mol) 4-Methoxy-2-thiophencarbonsäurechlorid in 20 ml abs. Chloroform versetzt. Bei Raumtemperatur werden 31,8 g (0,12 mol) Zinn(IV)chlorid innerhalb von 10 min zugetropft.Nach zwei Stunden Rühren bei 25°C wird das Reaktionsgemisch auf ein Gemisch von 150 ml Eis,15,0 ml conc. Salzsäure und 50,0 ml Wasser gegossen.

Die wäßrige Phase wird dreimal mit insgesamt 300ml Methylenchlorid extrahiert. Die vereinten organischen Phasen werden mit 100 ml ges.Natriumhydrogencarbonatlösung gewaschen, getrocknet und eingedampft. Es werden 29,4 g braunes Öl erhalten, das säulenchromatographisch gereinigt wird (400g Kieselgel,

Eluens: Petrolether:Benzol = 3:1). Die gesammelten Reinfraktionen werden kurzwegdestilliert.
Ausbeute: 9,12g goldgelbes, zähes Öl (33,3% d.Th.)
Siedepunkt: 140 - 150°C/0,007 mbar
Mikroelementaranalyse: $C_{18}H_{18}O_3S$ (314,41)

| | C | H |
|---|---|---|
| berechnet: | 68,76 | 5,77 |
| gefunden: | 68.81 | 5.74 |

$^1$H-NMR: ($CDCl_3$)
δ (ppm): 7,53 - 7,18 (m, 5H, Bz-H und Th-$H_3$); 6,77 (d, 1H, Th-$H_5$); 3,80 (s, 3H, -OC$\underline{H}_3$); 2,79 (t, 2H, Ar-C$\underline{H}_2$-); 1,97 - 1,15 (m, 4H, -C$\underline{H}_2$-C$\underline{H}_2$-); 0,90 (t, 3H, -C$\underline{H}_3$).

[3-(2-Butyl-benzo[b]furanyl)]-(4-hydroxy-2-thienyl)-methanon

8,54g (27,0 mmol) [3-(2-Butyl-benzo[b]furanyl)]-(4-methoxy-2-thienyl)-methanon werden in 100 ml abs. Chloroform gelöst und auf -10°C gekühlt. Bei einer Temperatur zwischen -10 und -5°C werden 22,5 g Bortribromid über 10 min zugetropft und danach das Reaktionsgemisch bei 0°C 30 min gerührt.

Danach wird auf eine Mischung von 100ml Methylenchlorid, 50g Eis und 25ml conc.Salzsäure gegossen, gerührt, die Phasen getrennt und die wäßrige Phase dreimal mit insgesamt 200ml Methylenchlorid extrahiert. Die vereinten organischen Phasen werden einmal mit 50ml 2n Salzsäure gewaschen, über Natriumsulfat getrocknet, mit Aktivkohle gerührt filtriert und eingedampft.
Ausbeute: 6,32g schwarzes, zähes Öl (77,9% d.Th.)
Mikroelementaranalyse: $C_{17}H_{16}O_3S$ (300,38)

| | C | H |
|---|---|---|
| berechnet: | 67,98 | 5,37 |
| gefunden: | 67,73 | 5,41 |

$^1$H-NMR: ($CDCl_3$)
δ(ppm): 7,61 - 7,15 (m, 5H, Bz-H und Th-$H_3$); 7,50 - 7,30 (s breit, 1H, -OH); 6,77 (d, 1H, Th-$H_5$); 2,93 (t, 2H, Ar-C$\underline{H}_2$-); 1,95 - 1,11 (m, 4H, -C$\underline{H}_2$-C$\underline{H}_2$-); 0,88 (t, 3H, -$CH_3$).

## Patentansprüche

### Patentansprüche für folgende Vertragsstaaten : AT,BE,CH,DE,FR,GB,IT, LI,LU,NL,SE

1. Thienyloxy-alkylamin-Derivate der allgemeinen Formel:

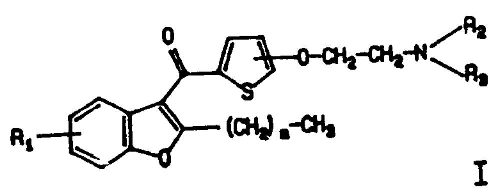

I ,

wobei -O-$CH_2$-$CH_2$-N($R_2$,$R_3$) in Position 4 oder 5 des Thiophenringes stehen kann und worin
$R_1$ Wasserstoff, Halogen, $CF_3$, Alkyl oder Alkoxy darstellt,
$R_2$ und $R_3$ gleich oder verschieden sind und jeweils Alkyl, Cycloalkyl, Alkenyl oder Alkinyl mit jeweils bis zu 8 C-Atomen bedeuten oder $R_2$ und $R_3$ zusammen mit dem sie verbindenden Stickstoffatom einen 5 bis 7-gliedrigen gesättigten Ring oder einen gesättigten heterocyclischen Ring bilden, der gegebenenfalls ein Sauerstoff- oder Stickstoffatom als weiteres Heteroatom im Ring enthalten kann, wobei ein zusätzliches Stickstoffatom durch einen Alkylrest mit 1 bis 3 C-Atomen substituiert sein kann, und

n eine ganze Zahl 1 bis 5 bedeutet,

und ihre pharmazeutisch verträglichen Säureadditionssalze.

2. Verbindungen der in Anspruch 1 definierten allgemeinen Formel I, worin $R_1$Wasserstoff und n = 3 bedeuten.

3. [3(2-Butyl-benzo[b]furanyl)]-[5-(2-diethylaminoethoxy)-2-thienyl]-methanon.

4. [3-(2-Butyl-benzo[b]furanyl)]-[5-(2-(1-pyrrolidinyl)-ethoxy)-2-thienyl]-methanon.

5. [3-(2-Butyl-benzo[b]furanyl)]-[4-(2-diethylaminoethoxy)-2-thienyl]-methanon.

6. [3-(2-Butyl-benzo[b]furanyl)]-[5-(2-diethylaminoethoxy)-2-thienyl]-methanon, Hydrochlorid.

7. [3-(2-Butyl-benzo[b]furanyl)]-[5-(2-(1-pyrrolidinyl)-ethoxy)-2-thienyl]-methanon, Hydrochlorid.

8. [3-(2-Butyl-benzo[b]furanyl)]-[4-(2-diethylaminoethoxy)-2-thienyl]-methanon, Hydrochlorid.

9. Verfahren zur Herstellung von Thienyloxyalkylamino-Derivaten der in Anspruch 1 definierten allgemeinen Formel I und ihren Säureadditionssalzen, dadurch gekennzeichnet, daß man eine Verbindung der allgemeinen Formel

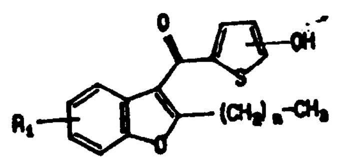

II,

wobei OH in Position 4 oder 5 des Thiophenringes stehen kann und in der $R_1$ und n die obige Bedeutung haben, mit einer Verbindung der allgemeinen Formel

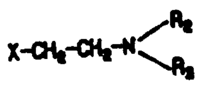

III,

in der $R_2$ und $R_3$ obige Bedeutung haben und X Chlor, Brom oder Jod bedeutet, in Gegenwart von mindestens einem Äquivalent einer starken Base in einem inerten organischen Lösungsmittel bei einer Temperatur von 60 bis 120°C umsetzt und die erhaltenen Basen erwünschtenfalls in ein pharmazeutisch verträgliches Salz überführt.

10. Pharmazeutische Präparate, enthaltend Verbindungen der allgemeinen Formel I nach Anspruch 1 oder ein Salz davon in Kombination mit üblichen galenischen Hilfs- und/oder Trägerstoffen.

11. Pharmazeutische Präparate, enthaltend Verbindungen der allgemeinen Formel I nach Anspruch 1 oder ein Salz davon in Kombination mit anderen therapeutisch wertvollen Wirkstoffen sowie üblichen galenischen Hilfs-und/oder Trägerstoffen.

12. Verbindungen nach Anspruch 1 zur Verwendung als Wirkstoffe für Arzneimittel zur Behandlung von Krankheiten des Herz-Kreislaufsystems.

13. Verbindungen nach Anspruch 1 zur Verwendung als Wirkstoffe für Antiarrhythmika.

**Patentansprüche für folgenden Vertragsstaat : ES**

1. Verfahren zur Herstellung von neuen Thienyloxy-alkylamin-Derivaten der allgemeinen Formel:

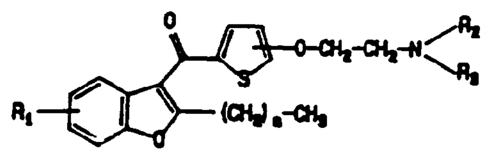

I,

wobei -O-$CH_2$-$CH_2$-N($R_2$,$R_3$) in Position 4 oder 5 des Thiophenringes stehen kann und worin

$R_1$ Wasserstoff, Halogen, $CF_3$, Alkyl oder Alkoxy darstellt,

$R_2$ und $R_3$ gleich oder verschieden sind und jeweils Alkyl, Cycloalkyl, Alkenyl oder Alkinyl mit jeweils bis zu 8 C-Atomen bedeuten oder $R_2$ und $R_3$ zusammen mit dem sie verbindenden Stickstoffatom einen 5 bis 7-gliedrigen gesättigten Ring oder einen gesättigten heterocyclischen Ring bilden, der gegebenenfalls ein Sauerstoff- oder Stickstoffatom als weiteres Heteroatom im Ring enthalten kann, wobei ein zusätzliches Stickstoffatom durch einen Alkylrest mit 1 bis 3 C-Atomen substituiert sein kann, und

n eine ganze Zahl 1 bis 5 bedeutet,

und ihren pharmazeutisch verträglichen Säureadditionssalzen, dadurch gekennzeichnet, daß man eine Verbindung der allgemeinen Formel

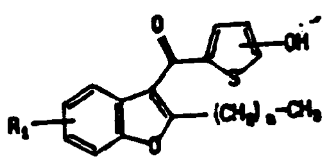

II,

wobei OH in Position 4 oder 5 des Thiophenringes stehen kann und in der $R_1$ und n die obige Bedeutung haben, mit einer Verbindung der allgemeinen Formel

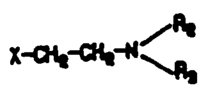

III,

in der $R_2$ und $R_3$ obige Bedeutung haben und X Chlor, Brom oder Jod bedeutet, in Gegenwart von mindestens einem Äquivalent einer starken Base in einem inerten organischen Lösungsmittel bei einer Temperatur von 60 bis 120°C umsetzt und die erhaltenen Basen erwünschtenfalls in ein pharmazeutisch verträgliches Salz überführt.

2. Verfahren nach Anspruch 1 zur Herstellung einer Verbindung der allgemeinen Formel I, worin $R_1$ Wasserstoff und n = 3 bedeuten, oder eines pharmazeutisch verträglichen Salzes davon.

3. Verfahren nach Anspruch 1 zur Herstellung von [3(2-Butyl-benzo[b] furanyl)]-[5-(2-diethylaminoethoxy)-2-thienyl] -methanon oder eines pharmazeutisch verträglichen Salzes davon.

4. Verfahren nach Anspruch 1 zur Herstellung von [3-(2-Butyl-benzo[b] furanyl)]-[5-(2-(1-pyrrolidinyl)-ethoxy)-2-thienyl]methanon oder eines pharmazeutisch verträglichen Salzes davon.

5. Verfahren nach Anspruch 1 zur Herstellung von [3-(2-Butyl-benzo[b] furanyl)]-[ 4-(2-diethylaminoethoxy)-2-thienyl] -methanon oder eines pharmazeutisch verträglichen Salzes davon.

6. Verfahren nach Anspruch 1 zur Herstellung von [3-(2-Butyl-benzo[b] furanyl)]-[5-(2-diethylaminoethoxy)-2-thienyl]-methanon, Hydrochlorid.

7. Verfahren nach Anspruch 1 zur Herstellung von [3-(2-Butyl-benzo[b] furanyl)]-[5-(2-1-pyrrolidinyl)-ethoxy)-2-thienyl]methanon, Hydrochlorid.

8. Verfahren nach Anspruch 1 zur Herstellung von [3-(2-Butyl-benzo[b] furanyl)]-[4-(2-diethylaminoethoxy)-2-thienyl]-methanon, Hydrochlorid.

9. Verfahren zur Herstellung von pharmazeutischen Präparaten, dadurch gekennzeichnet, daß man Verbindungen der allgemeinen Formel I oder ein Salz davon mit üblichen galenischen Hilfs- und/oder Trägerstoffen vermischt.

10. Verfahren zur Herstellung von pharmazeutischen Präparaten, dadurch gekennzeichnet, daß man Verbindungen der allgemeinen Formel I oder ein Salz davon mit anderen therapeutisch wertvollen Wirkstoffen sowie mit üblichen galenischen Hilfs- und/oder Trägerstoffen vermischt.

11. Verwendung von Verbindungen der allgemeinen Formel I als Wirkstoffe für Arzneimittel zur Behandlung von Krankheiten des Herz-Kreislaufsystems.

12. Verwendung von Verbindungen der allgemeinen Formel I als Wirkstoffe für Antiarrhythmika.

**Patentansprüche for folgenden Vertragsstaat : GR**

1. Thienyloxy-alkylamin-Derivate der allgemeinen Formel:

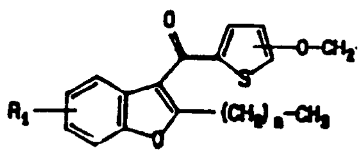

I,

wobei -O-$CH_2$-$CH_2$-N($R_2$,$R_3$) in Position 4 oder 5 des Thiophenringes stehen kann und worin
$R_1$ Wasserstoff, Halogen, $CF_3$, Alkyl oder Alkoxy darstellt,
$R_2$ und $R_3$ gleich oder verschieden sind und jeweils Alkyl, Cycloalkyl, Alkenyl oder Alkinyl mit jeweils bis zu 8 C-Atomen bedeuten oder $R_2$ und $R_3$ zusammen mit dem sie verbindenden Stickstoffatom einen 5 bis 7-gliedrigen gesättigten Ring oder einen gesättigten heterocyclischen Ring bilden, der gegebenenfalls ein Sauerstoff- oder Stickstoffatom als weiteres Heteroatom im Ring enthalten kann, wobei ein zusätzliches Stickstoffatom durch einen Alkyrest mit 1 bis 3 C-Atomen substituiert sein kann, und
n eine ganze Zahl 1 bis 5 bedeutet,
und ihre pharmazeutisch verträglichen Säureadditionssalze.

2. Verbindungen der in Anspruch 1 definierten allgemeinen Formel I, worin $R_1$ Wasserstoff und n = 3 bedeuten.

3. [3(2-Butyl-benzo[b]furanyl)]-[5-(2-diethylaminoethoxy)-2-thienyl]-methanon.

4. [ 3-(2-Butyl-benzo[b]furanyl)]- [ 5-(2-(1-pyrrolidinyl-ethoxy)-2-thienyl]-methanon.

5. [3-(2-Butyl-benzo[b]furanyl)]-[4-(2-diethylaminoethoxy)-2-thienyl]-methanon.

6. [3-(2-Butyl-benzo[b]furanyl)]-[5-(2-diethylaminoethoxy-2-thienyl] methanon, Hydrochlorid.

7. [ 3-(2-Butyl-benzo[b]furanyl)] [5-(2-(1-pyrrolidinyl-ethoxy)-2-thienyl] methanon, Hydrochlorid.

8. [ 3-(2-Butyl-benzo[b]furanyl)]-[4-(2-diethylaminoethoxy)-2-thienyl] methanon, Hydrochlorid.

9. Verfahren zur Herstellung von Thienyloxyalkylamino-Derivaten der in Anspruch 1 definierten allgemeinen Formel I und ihren Säureadditionssalzen, dadurch gekennzeichnet, daß man eine Verbindung der allgemeinen Formel

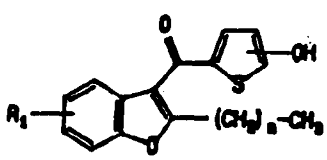

II,

wobei OH in Position 4 oder 5 des Thiophenringes stehen kann und in der $R_1$ und n die obige Bedeutung haben, mit einer Verbindung der allgemeinen Formel

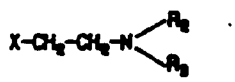

III,

in der $R_2$ und $R_3$ obige Bedeutung haben und X Chlor, Brom oder Jod bedeutet, in Gegenwart von mindestens einem Äquivalent einer starken Base in einem inerten organischen Lösungsmittel bei einer Temperatur von 60 bis 120°C umsetzt und die erhaltenen Basen erwünschtenfalls in ein pharmazeutisch verträgliches Salz überführt.

**10.** Verfahren zur Herstellung von pharmazeutischen Präparaten, dadurch gekennzeichnet, daß man Verbindungen der allgemeinen Formel I oder ein Salz davon mit üblichen galenischen Hilfs- und/oder Trägerstoffen vermischt.

**11.** Verfahren zur Herstellung von pharmazeutischen Präparaten, dadurch gekennzeichnet, daß man Verbindungen der allgemeinen Formel I oder ein Salz davon mit anderen therapeutisch wertvollen Wirkstoffen sowie mit üblichen galenischen Hilfs- und/oder Trägerstoffen vermischt.

**12.** Verwendung von Verbindungen der allgemeinen Formel I als Wirkstoffe für Arzneimittel zur Behandlung von Krankheiten des Herz-Kreislaufsystems.

**13.** Verwendung von Verbindungen der allgemeinen Formel I als Wirkstoffe für Antiarrhythmika.

## Claims

**Claims for the following Contracting States : AT,BE,CH,DE,FR,GB,IT,LI, LU,NL,SE**

**1.** Thienyloxyalkylamine derivatives of the general formula:

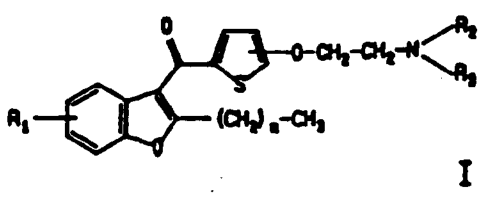

I

wherein $-O-CH_2-CH_2-NR_2R_3$ is in position 4 or 5 of the thiophene ring;

$R_1$ is hydrogen, halogen, $CF_3$, alkyl or alkoxy;

$R_2$ and $R_3$ are the same or different and are each alkyl, cycloalkyl, alkenyl or alkynyl each having up to 8 atoms, or $NR_2R_3$ is a 5 to 7-membered saturated heterocyclic ring optionally containing a further hetero atom which is oxygen or nitrogen optionally substituted by an alkyl group having 1 to 3 C atoms; and

n is an integer of 1 to 5;

and their pharmaceutically-acceptable acid addition salts.

**2.** Compounds of general formula I as defined in claim 1, wherein $R_1$ is hydrogen and n = 3.

**3.** [3-(2-Butylbenzo[b]furanyl)][5-(2-diethylamino-ethoxy)-2-thienyl]methanone.

**4.** [3-(2-Butylbenzo[b]furanyl)][5-(2-(1-pyrrolidinyl)-ethoxy)-2-thienyl]methanone.

**5.** [3-(2-Butylbenzo[b]furanyl)][4-(2-diethylamino-ethoxy)-2-thienyl]methanone.

**6.** [3-(2-Butylbenzo[b]furanyl)][5-(2-diethylamino-ethoxy)-2-thienyl]methanone hydrochloride.

7. [3-(2-Butylbenzo[b]furanyl)] [5-(2-(1-pyrrolidinyl)-ethoxy)-2-thienyl]methanone hydrochloride.

8. [3-(2-Butylbenzo[b]furanyl)] [4-(2-diethylamino-ethoxy)-2-thienyl]methanone hydrochloride.

9. Process for the preparation of thienyloxyalkylamino derivatives of general formula I as defined in claim 1 and their acid addition salts, in which a compound of the general formula

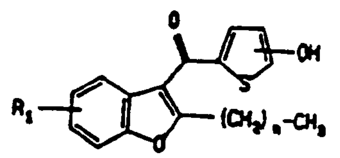

II

wherein OH is in the 4 or 5-position of the thiophene ring and $R_1$ and n are as defined above, is reacted with a compound of the general formula

$$X - CH_2 - CH_2NR_2R_3 \quad III$$

wherein $R_2$ and $R_3$ are as defined above and X is Cl, Br, or I, in the presence of at least one equivalent of a strong base, in an inert organic solvent, at a temperature from 60 to 120°C, and, optionally, converting the resultant base of formula I into the acid addition salt.

10. Pharmaceutical preparations comprising compounds of general formula I according to claim 1 or a salt thereof in combination with conventional galenic additive and/or carrier materials.

11. Pharmaceutical preparations comprising compounds of general formula I according to claim 1 or a salt thereof in combination with other therapeutically-valuable agents as well as conventional galenic additives and/or carrier materials.

12. Compounds according to claim 1 for use as agents for medicaments for the treatment of circulatory problems.

13. Compounds according to claim 1 for use as agents for anti-arrhythmica.

**Claims for the following Contracting State : ES**

1. Process for the preparation of thienyloxyalkylamine derivatives of the general formula:

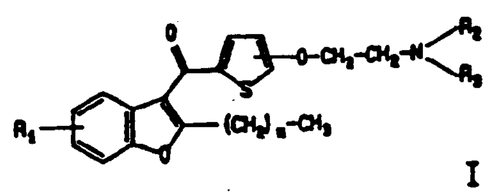

I

wherein $-O-CH_2-CH_2-NR_2R_3$ is in position 4 or 5 of the thiophene ring;

$R_1$ is hydrogen, halogen, $CF_3$, alkyl or alkoxy;

$R_2$ and $R_3$ are the same or different and are each alkyl, cycloalkyl, alkenyl or alkynyl each having up to 8 C atoms, or $NR_2R_3$ is a 5 to 7-membered saturated heterocyclic ring optionally containing a further hetero atom which is oxygen or nitrogen optionally substituted by an alkyl group having 1 to 3 C atoms; and

n is an integer of 1 to 5;

and their pharmaceutically-acceptable acid addition salts, in which a compound of the general formula

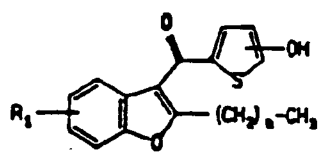

II

wherein OH is in the 4 or 5-position of the thiophene ring and $R_1$ and n are as defined above, is reacted with a compound of the general formula

$$X - CH_2 - CH_2NR_2R_3 \quad III$$

wherein $R_2$ and $R_3$ are as defined above and X is Cl, Br, or I, in the presence of at least one equivalent of a strong base, in an inert organic solvent, at a temperature from 60 to 120°C, and, optionally, converting the resultant base of formula I into the acid addition salt.

2. Process according to claim 1 for preparing a compound of general formula I, wherein $R_1$ is hydrogen and n = 3, or a pharmaceutically-acceptable acid addition salt thereof.

3. Process according to claim 1 for preparing [3-(2-butylbenzo[b] furanyl)]-[5-(2-diethylaminoethoxy)-2-thienyl]methanone or a pharmaceutically-acceptable acid addition salt thereof.

4. Process according to claim 1 for preparing [3-(2-butylbenzo[b] furanyl)]-[5-(2-(1-pyrrolidinyl)ethoxy)-2-thienyl]methanone, or a pharmaceutically-acceptable acid addition salt thereof.

5. Process according to claim 1 for preparing [ 3-(2-butylbenzo[b] furanyl)]-[4-(2-diethylaminoethoxy]-2-thienyl]methanone, or a pharmaceutically-acceptable acid addition salt thereof.

6. Process according to claim 1 for preparing [ 3-(2-Butylbenzo[b] furanyl)]-[5-(2-diethylaminoethoxy)-2-thienyl]methanone hydrochloride.

7. Process according to claim 1 for preparing [ 3-(2-butylbenzo[b] furanyl)]-[5-(2-(1-pyrrolidinyl)ethoxy)-2-thienyl]methanone hydrochloride.

8. Process according to claim 1 for preparing [ 3-(2-butylbenzo[b] furanyl)]-[4-(2-diethylaminoethoxy)-2-thienyl]methanone hydrochloride.

9. Process of preparing pharmaceutical preparations, comprising admixing compounds of general formula I or a salt thereof with conventional galenic additive and/or carrier materials.

10. Process of preparing pharmaceutical preparations, comprising admixing compounds of general formula I or a salt thereof with other therapeutically-valuable agents as well as conventional galenic additive and/or carrier materials.

11. Use of compounds of general formula I as agents for medicaments for the treatment of circulatory problems.

12. Use of compounds of general formula I as agents for anti-arrhythmica.

**Claims for the following Contracting State : GR**

1. Thienyloxyalkylamine derivatives of the general formula:

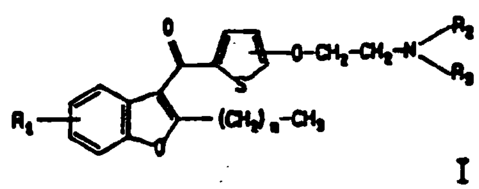

I

wherein $-O-CH_2-CH_2-NR_2R_3$ is in position 4 or 5 of the thiophene ring;

$R_1$ is hydrogen, halogen, $CF_3$, alkyl or alkoxy;

$R_2$ and $R_3$ are the same or different and are each alkyl, cycloalkyl, alkenyl or alkynyl each having up to 8 C atoms, or $NR_2R_3$ is a 5 to 7-membered saturated heterocyclic ring optionally containing a further hetero atom which is oxygen or nitrogen optionally substituted by an alkyl group having 1 to 3 C atoms; and

n is an integer of 1 to 5;

and their pharmaceutically-acceptable acid addition salts.

2. Compounds of general formula I as defined in claim 1, wherein $R_1$ is hydrogen and n = 3.

3. [3-(2-butylbenzo[b]furanyl)][5-(2-diethylamino-ethoxy)-2-thienyl]methanone.

4. [3-(2-butylbenzo[b]furanyl)][5-(2-(1-pyrrolidinyl)-ethoxy)-2-thienyl]methanone.

5. [3-(2-butylbenzo[b]furanyl)][4-(2-diethylamino-ethoxy)-2-thienyl]methanone.

6. [3-(2-butylbenzo[b]furanyl)][5-(2-diethylamino-ethoxy)-2-thienyl]methanone hydrochloride.

7. [3-(2-butylbenzo[b]furanyl)][5-(2-(1-pyrrolidinyl)-ethoxy)-2-thienyl]methanone hydrochloride.

8. [3-(2-butylbenzo[b]furanyl)][4-(2-diethylamino-ethoxy)-2-thienyl]methanone hydrochloride.

9. Process for the preparation of thienyloxyalkylamino derivatives of general formula I as defined in claim 1 and their acid addition salts, in which a compound of the general formula

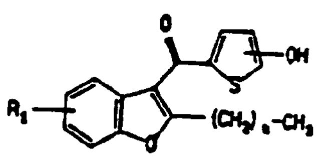

II

wherein OH is in the 4 or 5-position of the thiophene ring and $R_1$ and n are as defined above, is reacted with a compound of the general formula

$$X - CH_2 - CH_2NR_2R_3 \quad III$$

wherein $R_2$ and $R_3$ are as defined above and X is Cl, Br, or I, in the presence of at least one equivalent of a strong base, in an inert organic solvent, at a temperature from 60 to 120°C, and, optionally, converting the resultant base of formula I into the acid addition salt.

10. Process of preparing pharmaceutical preparations, comprising admixing compounds of general formula I or a salt thereof with conventional galenic additive and/or carrier materials.

11. Process of preparing pharmaceutical preparations, comprising admixing compounds of general formula I or a salt thereof with other therapeutically-valuable agents as well as conventional galenic additives and/or carrier materials.

12. Use of compounds of general formula I as agents for medicaments for the treatment of circulatory problems.

13. Use of compounds of general formula I as agents for anti-arrhythmica.

## Revendications

### Revendications pour les Etats Contractants Suivants : AT,BE,CH,DE,FR, GB,IT,LI,LU,NL,SE

1. Dérivés de thiényloxy-alkylamines de formule générale :

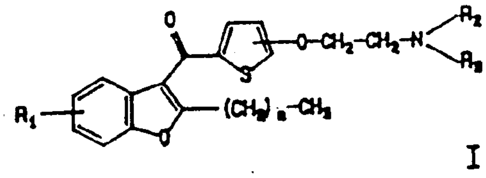

I ,

dans laquelle $-O-CH_2-CH_2-N(R_2,R_3)$ peut être en position 4 ou 5 du cycle thiophénique, et dans laquelle

$R_1$ représente un atome d'hydrogène, d'halogène, un groupe $CF_3$, alkyle ou alcoxy,

$R_2$ et $R_3$ sont identiques ou différents et représentent chacun un groupe alkyle, cycloalkyle, alcényle ou alcinyle ayant chacun jusqu'à 8 atomes de carbone, ou $R_2$ et $R_3$ forment, conjointement avec l'atome d'azote qui les porte, un cycle saturé comportant 5 à 7 chaînons, ou un noyau hétérocyclique saturé, qui peut contenir éventuellement un atome d'oxygène ou d'azote comme autre hétéroatome du cycle, un atome d'azote supplémentaire pouvant être substitué par un groupe alkyle comportant 1 à 3 atomes de carbone, et

n représente un nombre entier de 1 à 5,

et leurs sels d'addition avec des acides pharmaceutiquement acceptables.

2. Composés de formule générale I définie dans la revendication 1, dans laquelle $R_1$ représente un atome d'hydrogène et n = 3.

3. [3-(2-butyl-benzo[b]furanyl)] -[5-(2-diéthylamino-éthoxy)-2-thiényl] méthanone.

4. [3-(2-butyl-benzo[b]furanyl)]-[5-(2-(1-pyrrolidinyl)-éthoxy)-2-thiényl]méthanone.

5. [3-(2-butyl-benzo[b]furanyl)]-[4-(2-diéthylamino-éthoxy)-2-thiényl] méthanone.

6. Chlorhydrate de [3-(2-butyl-benzo[b]furanyl)]-[5-(2-diéthylaminoéthoxy)-2-thiényl]méthanone.

7. Chlorhydrate de [3-(2-butyl-benzo[b]furanyl)]-[5-(2-(1-pyrrolidinyl)-éthoxy)-2-thiényl]méthanone.

8. Chlorhydrate de [3-(2-butyl-benzo[b]furanyl)]-[4-(2-diéthylaminoéthoxy)-2-thiényl]méthanone.

9. Procédé pour la préparation de dérivés de thiényloxyalkylamines de formule générale I définie dans la revendication 1, et de leurs sels d'addition avec des acides, caractérisé en ce que l'on fait réagir un composé de formule générale

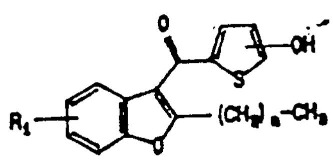

II ,

dans laquelle OH peut être en position 4 ou 5 du cycle thiophénique et dans laquelle $R_1$ et n ont les significations mentionnées ci-dessus, avec un composé de formule générale

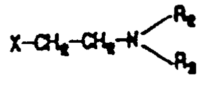

III ,

dans laquelle $R_2$ et $R_3$ ont les significations mentionnées ci-dessus et X représente un atome de chlore, de brome ou d'iode, en présence d'au moins un équivalent d'une base forte dans un solvant organique

inerte, à une température de 60 à 120°C, et l'on transforme éventuellement la base obtenue en un sel pharmaceutiquement acceptable.

**10.** Préparations pharmaceutiques, contenant des composés de formule générale I selon la revendication 1 ou un de leurs sels, en combinaison avec des adjuvants et/ou supports galéniques usuels.

**11.** Préparations pharmaceutiques, contenant des composés de formule générale I selon la revendication 1 ou un de leurs sels, en combinaison avec d'autres substances thérapeutiquement actives ainsi qu'avec des adjuvants et/ou supports galéniques usuels.

**12.** Composés selon la revendication 1, destinés à l'utilisation comme substances actives de médicaments pour le traitement des maladies cardiovasculaires.

**13.** Composés selon la revendication 1, destinés à l'utilisation comme substances actives de médicaments antiarythmiques.

**Revendications pour l'Etat Contractant Suivant : ES**

**1.** Procédé pour la préparation de nouveaux dérivés de thiényloxy-alkylamines de formule générale :

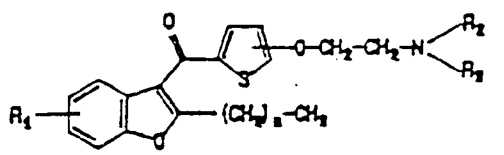

I,

dans laquelle $-O-CH_2-CH_2-N(R_2,R_3)$ peut être en position 4 ou 5 du cycle thiophénique, et dans laquelle

$R_1$ représente un atome d'hydrogène, d'halogène, un groupe $CF_3$, alkyle ou alcoxy,

$R_2$ et $R_3$ sont identiques ou différents et représentent chacun un groupe alkyle, cycloalkyle, alcényle ou alcinyle ayant chacun jusqu'à 8 atomes de carbone, ou $R_2$ et $R_3$ forment, conjointement avec l'atome d'azote qui les porte, un cycle saturé comportant 5 à 7 chaînons, ou un noyau hétérocyclique saturé, qui peut contenir éventuellement un atome d'oxygène ou d'azote comme autre hétéroatome du cycle, un atome d'azote supplémentaire pouvant être substitué par un groupe alkyle comportant 1 à 3 atomes de carbone, et

n représente un nombre entier de 1 à 5,

et leurs sels d'addition avec des acides pharmaceutiquement acceptables, caractérisé en ce que l'on fait réagir un composé de formule générale

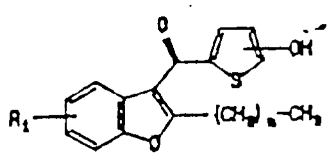

II,

dans laquelle OH peut être en position 4 ou 5 du cycle thiophénique et dans laquelle $R_1$ et n ont les significations mentionnées ci-dessus, avec un composé de formule générale

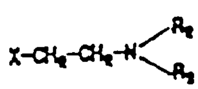

III,

dans laquelle $R_2$ et $R_3$ ont les significations mentionnées ci-dessus et X représente un atome de chlore, de brome ou d'iode, en présence d'au moins un équivalent d'une base forte dans un solvant organique inerte, à une température de 60 à 120°C, et l'on transforme éventuellement la base obtenue en un sel pharmaceutiquement acceptable.

2. Procédé selon la revendication 1, pour la préparation d'un composé de formule générale 1, dans laquelle $R_1$ représente un atome d'hydrogène et n = 3, ou d'un de ses sels pharmaceutiquement acceptables.

3. Procédé selon la revendication 1, pour la préparation de la [3-(2-butyl-benzo[b]furanyl)]-[5-(2-diéthylaminoéthoxy)-2-thiényl] méthanone, ou d'un de ses sels pharmaceutiquement acceptables.

4. Procédé selon la revendication 1, pour la préparation de la [3-(2-butyl-benzo[b]furanyl)]-[5-(2-1-pyrolidinyl)éthoxy)-2-thiényl]méthanone, ou d'un de ses sels pharmaceutiquement acceptables.

5. Procédé selon la revendication 1, pour la préparation de la [3-(2-butyl-benzo[b]furanyl)]-[4-(2-diéthylaminoéthoxy)-2-thiényl]méthanone, ou d'un de ses sels pharmaceutiquement acceptables.

6. Procédé selon la revendication 1, pour la préparation du chlorhydrate de [3-(2-butyl-benzo[b]furanyl)]-[5-(2-diéthylaminoéthoxy)-2-thiényl] méthanone.

7. Procédé selon la revendication 1, pour la préparation du chlorhydrate de [3-(2-butyl-benzo[b]furanyl)]- [5-(2-(1-pyrrolidinyl)-éthoxy)-2-thiényl]méthanone.

8. Procédé selon la revendication 1, pour la préparation du chlorhydrate de [3-(2-butyl-benzo[b]furanyl)]-[4-(2-diéthylaminoéthoxy)-2-thiényl]méthanone.

9. Procédé pour l'obtention de préparations pharmaceutiques, caractérisé en ce que l'on mélange des composés de formule générale I ou leurs sels avec des adjuvants et/ou supports galéniques usuels.

10. Procédé d'obtention de préparations pharmaceutiques, caractérisé en ce que l'on mélange des composés de formule générale I ou un de leurs sels avec d'autres substances thérapeutiquement actives ainsi que des adjuvants et/ou supports galéniques usuels.

11. Utilisation de composés de formule générale I comme substances actives pour des médicaments destinés au traitement des maladies cardiovasculaires.

12. Utilisation de composés de formule générale I comme substances actives de médicaments antiarythmiques.

**Revendications pour l'Etat Contractant Suivant : GR**

1. Dérivés de thiényloxy-alkylamines de formule générale :

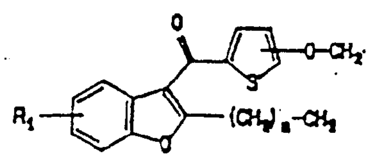

I,

dans laquelle $-O-CH_2-CH_2-N(R_2,R_3)$ peut être en position 4 ou 5 du cycle thiophénique, et dans laquelle

$R_1$ représente un atome d'hydrogène, d'halogène, un groupe $CF_3$, alkyle ou alcoxy,

$R_2$ et $R_3$ sont identiques ou différents et représentent chacun un groupe alkyle, cycloalkyle, alcényle ou alcinyle ayant chacun jusqu'à 8 atomes de carbone, ou $R_2$ et $R_3$ forment, conjointement avec l'atome d'azote qui les porte, un cycle saturé comportant 5 à 7 chaînons, ou un noyau hétérocyclique saturé, qui peut contenir éventuellement un atome d'oxygène ou d'azote comme autre hétéroatome du cycle, un atome d'azote supplémentaire pouvant être substitué par un groupe alkyle comportant 1 à 3 atomes de carbone, et

n représente un nombre entier de 1 à 5,

et leurs sels d'addition avec des acides pharmaceutiquement acceptables.

2. Composés de formule générale I définie dans la revendication 1, dans laquelle $R_1$ représente un atome d'hydrogène et n = 3

3. [3-(2-butyl-benzo[b]furanyl)]-[5-(2-diéthylaminoéthoxy)-2-thiényl]méthanone.

4. [3-(2-butyl-benzo[b]furanyl)]-[5-(2-(1-pyrrolidinyl)-ethoxy)-2-thiényl] méthanone.

5. [3-(2-butyl-benzo[b]furanyl)]-[4-(2-diéthylaminoéthoxy)-2-thiényl] méthanone.

6. Chlorhydrate de [3-(2-butyl-benzo[b]furanyl)]-[5-(2-diéthylaminoéthoxy)-2-thiényl]méthanone.

7. Chlorhydrate de [3-(2-butyl-benzo[b]furanyl)]-[5-(2-(1-pyrrolidinyl)-éthoxy)-2-thiényl]méthanone.

8. Chlorhydrate de [3-(2-butyl-benzo[b]furanyl)]-[4-(2-diéthylaminoéthoxy)-2-thiényl]méthanone.

9. Procédé pour la préparation de dérivés de thiényloxy-alkylamines de formule générale définie dans la revendication 1, et de leurs sels d'addition avec des acides, caractérisé en ce que l'on fait réagir un composé de formule générale

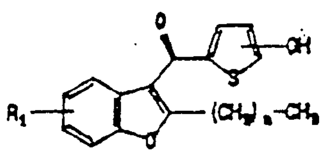

II,

dans laquelle OH peut être en position 4 ou 5 du cycle thiophénique et dans laquelle $R_1$ et n ont les significations mentionnées ci-dessus, avec un composé de formule générale

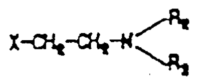

III,

dans laquelle $R_2$ et $R_3$ ont les significations mentionnées ci-dessus et X représente un atome de chlore, de brome ou d'iode, en présence d'au moins un équivalent d'une base forte dans un solvant organique inerte, à une température de 60 à 120°C, et l'on transforme éventuellement la base obtenue en un sel pharmaceutiquement acceptable.

10. Procédé pour l'obtention de préparations pharmaceutiques, caractérisé en ce que l'on mélange des composés de formule générale I ou leurs sels avec des adjuvants et/ou supports galéniques usuels.

11. Procédé pour l'obtention de préparations pharmaceutiques, caractérisé en ce que l'on mélange des composés de formule générale I ou un de leurs sels avec d'autres substances thérapeutiquement actives ainsi que des adjuvants et/ou supports galéniques usuels.

12. Utilisation de composés de formule générale I comme substances actives pour des médicaments destinés au traitement des maladies cardiovasculaires.

13. Utilisation de composés de formule générale I comme substances actives de médicaments antiarythmiques.